# EUROPEAN PATENT SPECIFICATION

(11) **EP 4 015 646 B1**
(45) Date of publication and mention of the grant of the patent: **27.12.2023**
(21) Application number: 20852389.4
(22) Date of filing: 11.08.2020
(51) Int. Cl.: C12Q 1/6806, C12Q 1/6869

(54) **NUCLEIC ACID SAMPLE PROCESSING METHOD, SEQUENCING METHOD AND KIT**
VERFAHREN ZUR VERABREITUNG VON NUKLEINSÄUREN, SEQUENZIERUNGSVERFAHREN UND KIT
PROCÉDÉ DE TRAITEMENT D'ÉCHANTILLON D'ACIDE NUCLÉIQUE, PROCÉDÉ DE SÉQUENÇAGE ET KIT

(30) Priority: 12.08.2019 CN 201910741127; 16.09.2019 CN 201910868866
(43) Date of publication of application: 22.06.2022
(73) Proprietor: GeneMind Biosciences Company Limited, Shenzhen, Guangdong (CN)
(72) Inventor: ZHANG, Meng, Shenzhen, Guangdong (CN); LIU, Lichun, Shenzhen, Guangdong (CN); FENG, Yan, Shenzhen, Guangdong (CN); LI, Gailing, Shenzhen, Guangdong (CN); LIN, Qunting, Shenzhen, Guangdong (CN); GAN, Guangli, Shenzhen, Guangdong (CN)
(74) Representative: Schiweck Weinzierl Koch Patentanwälte Partnerschaft mbB
(86) International application number: PCT/CN2020/108420
(87) International publication number: WO 2021/027810

(56) References cited:
- WO-A1-2018/090373
- WO-A1-2018/144363
- WO-A1-2018/233236
- WO-A1-2019/023924
- WO-A2-2018/191702
- CN-A- 108 251 504
- CN-A- 108 330 546
- CN-A- 108 796 061
- CN-A- 109 385 469

## Description

### CROSS REFERENCE TO RELATED APPLICATIONS

The present application claims priority and benefits to Chinese Patent Application No. CN201910741127.1 filed with China National Intellectual Property Administration on Aug. 12, 2019 and Chinese Patent Application No. CN201910868866.7 filed with China National Intellectual Property Administration on Sep. 16, 2019, each of which is incorporated herein by reference in its entirety.

### TECHNICAL FIELD

The present application relates to the field of biomacromolecule detection and analysis, and in particular to a method for processing a nucleic acid sample, a sequencing method and a kit.

### BACKGROUND

High-throughput sequencing, including next-generation sequencing, single-molecule sequencing, etc., involves the processing of a nucleic acid sample before on-line sequencing. In particular, the nucleic acid sequence determination based on chip assay requires the processing of the nucleic acid (template) to be tested, so that it can be ligated to the chip for sequencing.

At present, commercially available sequencing platforms based on chip assay are generally sold with a compatible chip, which has a specific sequence (probe).The template generally needs to be processed by various processes to contain the specific sequence or at least part of a sequence matching the specific sequence, so that the template can be bound to the chip. The various processes, for a long nucleic acid fragment, such as genomic DNA, in particular, generally involve mechanical or enzymatic cleavage of the nucleic acid to obtain a nucleic acid fragment, and repairs the nucleic acid fragment, such as end filling-in and/or providing a specific sticky end, so that the nucleic acid fragment can be ligated to the specific sequence (adapter) to obtain a sequencing library. WO 2018/090373 A1 (MGI TECH CO LTD [CN]) and WO 2018/233236 A1 (BERRY GENOMICS CO LTD [CN]) describe methods and kits for constructing sequencing libraries.

At present, there are various commercially available kits for the steps of the processing of nucleic acids before on-line sequencing, for example, a fragmentation kit, an optional dA-tailing kit for end repair, an adapter kit, an adapter ligation kit and a purification kit for each step, as well as kits containing specific reagents for detecting and/or removing interference from previous reaction systems and subsequent reactions, etc. However, generally, no specific formula is disclosed about the commercially available kit, including the species, characteristics and concentration of an enzyme, and specific reaction solution configuration, etc.

It is necessary to develop and improve a quick and simple method for processing nucleic acids before sequencing, as well as related solution systems.

### SUMMARY

The present application provides a method for processing a DNA sample, wherein a plurality of enzymes are mixed in the same reaction system, so that DNA fragmentation, end repair and dA-tailing can be implemented in one step, thereby enabling a simple and quick operation, saving the processing time before on-line sequencing, and facilitating the industrialization. In addition, the present application provides a method for sequencing a sample, comprising after processing a DNA sample using the method for processing a DNA sample disclosed herein, performing next- and/or third-generation sequencing by probe capture or hybridization. The present application further provides a kit for processing and sequencing a DNA sample, wherein End Prep Mix is formed by mixing a plurality of enzymes and buffers, leading to a simplified operation.

In one aspect, the present application provides a method for processing a DNA sample, which comprises fragmenting, end-repairing and dA-tailing the DNA in a first pre-mixed system to obtain a first product, wherein the first pre-mixed system comprises a pre-mixed enzyme comprising a DNA-fragmentation enzyme, a polynucleotide kinase, and a DNA polymerase.

It could be understood that the DNA-fragmentation enzyme is used in the above method to fragment the DNA sample to a desired size. In the embodiments of the present application, the DNA-fragmentation enzyme may be selected from T7 endodeoxyribonuclease I, *Yersinia pestis* bacteriophage phiA1122 endonuclease, bacteriophage PhiYe03-12 endonuclease, bacteriophage T3 endonuclease, bacteriophage T3 endodeoxyribonuclease, *Pseudomonas* bacteriophage gh-1 endonuclease, *Pseudomonas putida* KT2440 endodeoxyribonuclease I and roseophage S101 RP endonuclease I, and available kits include Tiangen DNA-fragmentation kit NG305-02, Qiagen CM0162 fragmentation enzyme kit and NEB (New England Biolabs) fragmentation enzyme, etc.

In one embodiment, the DNA in the method has a size of no less than 5 Kbp, and/or the first product obtained after fragmentation has a size of 100-400 bp. Based on the sequencing length limit of a sequencer, In one embodiment, the screened first product has a size of 100-300 bp.

In one embodiment, in the method, the DNA in the first pre-mixed system has a content or amount of 10-60 ng, preferably 15-50 ng. The DNA content is directly related to the size of the reaction system, the amount of the enzymes, and the library size needed. If the DNA content is too low, the size of the constructed library would be small, and the data obtained by sequencing cannot meet the requirements; however, if the DNA content is too high, in a certain solution system, the DNA cannot be sufficiently reacted, for example, cannot be sufficiently fragmented, and/or ligated to adapters, resulting in many ineffective fragments in the constructed library. After many tests, the inventor found that the DNA content is set to 15ng~50ng, which can make the fragmentation and other reactions(for example, at 32°C) to be carried out effectively, and the library constructed with this amount of DNA can meet the needs of on-machine sequencing.

In one embodiment, the first pre-mixed system further comprises Mg²⁺, wherein the Mg²⁺ has a concentration of 60-100 mM, preferably 60-75 mM. In general, different enzymes have different requirements for Mg²⁺, and the mixing of a plurality of enzymes at a same solution makes it difficult for each enzyme to function under its optimum or superior conditions. In one embodiment of the present application, the first pre-mixed system can be diluted to 5 times the original volume before reaction, that is, in the diluted overall reaction system, the Mg²⁺ concentration optimized for mixed enzyme reaction is 12-15 mM, which is significantly higher than the concentration of about 6mM conventionally used to catalyze reactions using each of these enzymes alone .

In one embodiment, in the first pre-mixed system, the amount of the DNA-fragmentation enzyme is at least 2 times the amount of the polynucleotide kinase, the amount corresponds to enzyme activity; that is, the size of enzyme activity (the amount of enzyme) can be expressed in enzyme activity unit (U); and the DNA polymerase could be screened from at least one of DNA polymerase I and Taq DNA polymerase, and preferably, the DNA polymerase consists of a Klenow fragment of DNA polymerase I, and Taq DNA polymerase. The polynucleotide kinase is T4 polynucleotide kinase.

In one embodiment, the ratio of the amount of the DNA-fragmentation enzyme to the polynucleotide kinase to the Klenow fragment of DNA polymerase I to Taq DNA polymerase in the first pre-mixed system is [16, 35]:[6, 10]:[1, 3]:[1, 5], and the ratio of the amount corresponds to the ratio of the enzyme activity; this ratio is obtained after many experiments and optimization, and the mixed enzyme in this ratio can efficiently process the sample and obtain the desired effect or result. In one embodiment of the present application, the ratio of the total enzyme activity of the DNA-fragmentation enzyme to the polynucleotide kinase to the Klenow fragment of DNA polymerase I to Taq DNA polymerase is 18:6:1:1, and the concentration of Taq DNA polymerase in the first pre-mixed system is (1/3 to 2/3) U/µL, wherein the activity unit of Taq DNA polymerase is defined as follows: the amount of the enzyme required to incorporate 10 nmol of dNTP into acid-insoluble precipitate at 37 °C within 30 min is defined as 1 activity unit (U); the activity unit of the Klenow fragment enzyme is defined as follows: the amount of the enzyme required to incorporate 10 nmol of dNTP into acid-insoluble precipitate at 37 °C within 30 min is defined as 1 activity unit (U); the activity unit of polynucleotide kinase is defined as follows: the amount of the enzyme required to incorporate 1 nmol of [γ-32P] ATP into acid-insoluble precipitate at 37 °C within 30 min is defined as 1 activity unit (U); the activity unit of DNA-fragmentation enzyme is defined as follows: in a 20 mL reaction system, the amount of the enzyme required to convert 1 µg of supercoiled pUC(AT) into the linear form (two strands being cut apart) or the nicked form (a single strand being cut apart) at 37 °C within 30 min is defined as 1 activity unit (LT).

In any of the above embodiments, the method for processing a DNA sample, based on tests and an optimized mixed enzyme system, multiple sample processing process, including DNA fragmentation, end repair and dA-tailing, are efficiently implemented in one step, and the processed nucleic acid obtained is suitable for being detected on various sequencing platforms; the method is simple and quick to implement, and can save the processing time before on-line sequencing, which is beneficial to industrialization; further, the product obtained by the processing can be directly used in the next step without separation from the reaction system or purification.

In one embodiment, the above method further comprises: providing at least one end of the first product with a predetermined sequence in a second pre-mixed system to obtain a second product, wherein the second pre-mixed system comprises the first pre-mixed system and the predetermined sequence.

In one embodiment, the second pre-mixed system further comprises a ligase, such as T4 DNA ligase.

In one embodiment, the predetermined sequence is a double-stranded DNA consisting of a first strand and a second strand that are complementary, and has at least one single-stranded end.

In one embodiment, the 5'-end of the first strand has a phosphate group, and the 3'-end of the second strand has no hydroxyl group; the 5'-end of the first strand is ligated to the first product, and after the ligation using this predetermined sequence, only the first strand is ligated to the first product; in a PCR-free library construction, the library constructed using this method can be directly used for sequence capture.

In one embodiment, 80 °C ≥ (Tm1 - Tm2) ≥ 10 °C and 90 °C ≥ Tm1 ≥ 50 °C, where Tm1 is a melting temperature of the first strand, and Tm2 is a melting temperature of the second strand. In one example, the Tm1 is 71 °C, and the Tm2 is 45.6 °C. The difference between the melting temperatures of the two strands is utilized to reduce the effect of the second strand on the capture efficiency during sequence capture.

In one embodiment, the predetermined sequence is selected from at least one of the following sequence combinations:
1) a sequence set forth in SEQ ID NO: 1 and a sequence set forth in SEQ ID NO: 2;
2) a sequence set forth in SEQ ID NO: 1 and a sequence set forth in SEQ ID NO: 3; and
3) a sequence set forth in SEQ ID NO: 4 and a sequence set forth in SEQ ID NO: 5.

Based on any of the above methods, the nucleic acid to be tested is provided with a predetermined sequence (also referred to herein as adapter) by ligation and/or polymerization. With the reaction system involved in any of the above methods and the designed adapter, a nucleic acid (sequencing library) to be tested containing a specific sequence can be rapidly and efficiently prepared, so as to meet the requirements for subsequent on-line sequencing.

In a second aspect, the present application provides a method for sequencing a DNA, which comprises:
processing the DNA sample using any of the method for processing a DNA sample described above to obtain a first product and a second product; and
linking the second product to a surface of a solid substrate and sequencing the second product to determine at least part of the sequence of the DNA.

In one embodiment, the second product carries an optically detectable label, such as a fluorescent molecule.

In one embodiment, the solid substrate has a probe on its surface, and the second product is linked to the surface of the solid substrate by the probe, which has a length of 20-80 nt. The solid substrate is selected from at least one of glass, plastic and magnetic beads.

In one embodiment, the predetermined sequence is a double-stranded DNA consisting of a first strand and a second strand that are complementary, and has a single-stranded end located on the first strand. Further, the first strand is completely/fully complementary to the probe, and its length is not longer than that of the probe. Further, Tm2 - 5 °C ≤ T ≤ Tm1 - 5 °C, where T is a reaction temperature for hybridizing the second product to the surface of the solid substrate, and is also a temperature for sequence capture of the probe, Tm1 is a melting temperature of the first strand, and Tm2 is a melting temperature of the second strand.

In one embodiment, the method for sequencing a DNA further comprising denaturing the second product before linking the second product to the surface of the solid substrate.

In the method for sequencing a DNA, a probe and/or reaction conditions meeting specific requirements are designed and screened, so that a sequencing library can be efficiently and stably linked to the surface of the solid substrate, thereby facilitating stable and efficient sequencing and obtaining template information; in addition, any of the methods has the advantages and technical features of the method in any of the above examples, which would not be repeated herein.

In a third aspect, the present application provides a kit comprising a first pre-mixed system for implementing the method for processing a DNA sample and the method for sequencing the DNA as described above. When the kit comprising the first pre-mixed system is used for processing a DNA sample, DNA fragmentation, end repair and dA-tailing can be implemented in one step thereby enabling a simple and quick operation, and saving the DNA processing time.

In one embodiment, the kit further comprises the second pre-mixed system described in any of the embodiments above.

In one embodiment, the kit further comprises a probe for implementing the method for sequencing a DNA, wherein a second product is linked to the surface of the solid substrate by the probe, which is a DNA sequence having a length of 20-80 nt.

In one embodiment, a predetermined sequence is a double-stranded DNA consisting of a first strand and a second strand that are complementary, and has a single-stranded end located on the first strand; and the probe is complementary to the first strand, and the length of the first strand is not longer than that of the probe.

Any of the above kits can be used to implement any of the above methods, so that any of the methods has the aforementioned advantages, which is beneficial to industrialization. Any of the kits can be used to process a nucleic acid before sequencing, optionally, to sequence the nucleic acid.

### BRIEF DESCRIPTION OF THE DRAWINGS

FIG. 1: labchip assay of the products obtained by adapter ligation of the 4 samples F4, F5, F6 and F7 in the Example ;
FIG. 2: labchip assay of the PCR products of the 4 samples F4, F5, F6 and F7 in the Example; and
FIG. 3: labchip assay of the products obtained by cleavage, end-repairing and dA-tailing of the 12 samples N5, N6, N13, N14, N21, N22, N23, N24, 05, 06, O11 and 012 in the Example.

### DETAILED DESCRIPTION

Unless otherwise specified, the terms used herein have the ordinary meanings known in the art to which the present application pertains.

The present application could be described below with reference to specific examples, which are intended to be illustrative only and are not to be construed as limiting the present application.

### Materials and reagents

Adapter 1 (D9-T): a double-stranded nucleic acid formed from two single-stranded nucleic acids (a first strand and a second strand), i.e., a double-stranded nucleic acid formed from a sequence set forth in SEQ ID NO: 1 and a sequence set forth in SEQ ID NO: 2, or a double-stranded nucleic acid formed from a sequence set forth in SEQ ID NO: 1 and a sequence set forth in SEQ ID NO: 3:
the first strand: 5'-AGATGTGTATAAGAGACAGT-3' (SEQ ID NO: 1)
the second strand:
   5'-ACTGTCTCTTATACACATCTGAGTGGAACTGGATGGTCGCAGGTATCAAGGATT-3' (SEQ ID NO: 2) or
   5'-CTGTCTCTTATACACATCTGAGTGGAACTGGATGGTCGCAGGTATCAAGGA-3' (SEQ ID NO: 3)

Adapter 2: a double-stranded nucleic acid formed from two single-stranded nucleic acids (a first strand and a second strand), e.g., a P5 and/or P7 adapter that matches some of ILLUMINA sequencing platforms; can be an adapter in commercially available kits, e.g., from Vazyme:
the first chain: 5'-ACACTCTTTCCCTACACGACGCTCTTCCGATC-s-T-3' (SEQ ID NO: 4)
the second chain: 5'-p-GATCGGAAGAGCACACGTCTGAACTCCAGTC-3' (SEQ ID NO: 5)
(-s- represents thio, and -p means phosphorylated)

If techniques or conditions are not specifically indicated in examples, conventional experimental conditions or conditions recommended in the manufacturer's manual are adopted. The reagents or instruments not provided with manufacturer are conventional and commercially available products.

### Examples

A method for preparing a kit is illustrated below.

The kit comprised a tube 1, which was a mixed system for end-repair (also referred to as End Prep Mix). In one case, tube 1 was a solution comprising a polynucleotide kinase and a plurality of DNA polymerases. In another case, tube 1 was a solution comprising a DNA-fragmentation enzyme, a polynucleotide kinase, and a plurality of DNA polymerases.

The DNA polymerase can fill-in the ends of nucleic acid fragments; herein, preferably, the screened DNA polymerase can be used for amplification, and also for repair and proofreading; for example, the DNA polymerase was screened from one or two of Taq DNA polymerase (simply as Taq enzyme) and Klenow fragment. The polynucleotide kinase can be selected from T4 polynucleotide kinase.

In one case, the DNA polymerase was a mixture of Taq enzyme and the Klenow fragment; the enzyme activity unit (U) ratio of Taq enzyme to the Klenow fragment to the polynucleotide kinase in tube 1 was 1:1:6, and the concentration of Taq enzyme in tube 1 was (1-2) U/µL. Herein, the activity units of the enzymes were defined as follows: the activity unit of Taq enzyme: the amount of the enzyme required to incorporate 10 nmol of dNTP into acid-insoluble precipitate at 37 °C within 30 min was defined as 1 activity unit (U); the activity unit of the Klenow fragment: the amount of the enzyme required to incorporate 10 nmol of dNTP into acid-insoluble precipitate at 37 °C within 30 min was defined as 1 activity unit (U); and the activity unit of polynucleotide kinase: the amount of th enzyme required to incorporate 1 nmol of [γ-32P] ATP into acid-insoluble precipitate at 37 °C within 30 min was defined as 1 activity unit (U).

In one case, tube 1 further comprised a DNA-fragmentation enzyme; a mixed enzyme solution A without the DNA-fragmentation enzyme was first prepared, and then the DNA-fragmentation enzyme was added to mixed enzyme solution A to obtain tube 1. For example, the DNA-fragmentation enzyme solution was mixed with mixed enzyme solution A in a volume ratio of 1:2, such that the enzyme activity unit (U) ratio of the DNA-fragmentation enzyme to Taq enzyme (namely Taq DNA polymerase) to the Klenow fragment to the polynucleotide kinase in tube 1 was 18:1:1:6, and the concentration of Taq enzyme in tube 1 was 2/3 of the concentration of Taq enzyme in mixed enzyme solution A. The DNA-fragmentation enzyme can be screened from Tiangen DNA-fragmentation kit NG305-02, Qiagen CM0162 fragmentation enzyme kit and NEB fragmentation enzyme. The Taq enzyme, Klenow and polynucleotide kinase in mixed enzyme solution A can be selected from corresponding products sold by NEB, Tiangen, Vazyme, etc. The activity unit of DNA-fragmentation enzyme was defined as follows: in a 20 mL reaction system, the amount of the enzyme required to convert 1 µg of supercoiled pUC(AT) into the linear form (two strands being cut apart) or the nicked form (a single strand being cut apart) at 37 °C within 30 min was defined as 1 activity unit (LT).

In the preparation of tube 1, enzyme solutions can be prepared or purchased separately, and then the separate enzyme solutions were mixed; alternatively, a mixed enzyme solution and a buffer solution can be prepared separately and then mixed, for example, in a volume ratio of 1:1, to obtain tube 1.

In one case, in the preparation of tube 1, enzyme solutions, including separate DNA-fragmentation enzyme, T4 polynucleotide kinase, Taq DNA polymerase and Klenow fragment kit, were purchased from manufactures separately, and the separate enzyme solutions in the purchased kits were mixed in a specific ratio, and then an additional MgCl₂ solution, for example, MgCl₂ of no less than 60 mM, was added to obtain tube 1.

In one case, the various enzymes in tube 1 were placed collectively in the following solution system: 10× buffer having the following specific components: 700 mM Tris-HCl pH 7.6, 120-150 mM MgCl₂, 50 mM DTT and 1 mM dNTPs. Herein, 1 mM dNTPs means that the concentration of each dNTP was 1 mM. The volume ratio of the total of various enzymes to the 10× buffer in tube 1 was 1:1, and the mixed system in tube 1 obtained was 5× End Prep Mix, i.e., when the mixed system in tube 1 was used for DNA fragmentation, end repair and dA-tailing, the volume ratio of the mixed system of tube 1 to the total reaction system was 1:5. The components and their concentrations in the buffer in tube 1 were as follows: 350 mM Tris-HCl pH 7.6, 60-75 mM MgCl₂, 25 mM DTT and 0.5 mM dNTPs. The concentration of Taq enzyme in tube 1 was (1/3-2/3) U/µL.

In some cases, the kit further comprised a tube 2, which was a mixed system for ligation, namely a ligation mix consisting of a DNA ligase and a ligase buffer system. The DNA Ligase may be Blunt/TA Ligase, e.g., T4 DNA Ligase. The components and their concentrations in the 2× ligation mix were as follows: T4 DNA ligase buffer: 80-100 mM Tris-HCl pH 7.8, 20 mM MgCl₂, 20 mM DTT, 10 mM ATP and 50 µg/mL BSA. The activity unit concentration of T4 DNA ligase was 60-100 U/µL. Different concentrations of ligation mix can be prepared as needed. The activity unit of T4 DNA ligase was defined as the amount of the enzyme required to ligate 50% of 6 µg λ DNA HindIII-digested product at 16 °C within 30 min, in 20 µL of the following reaction system: 50 mM Tris-HCl pH 7.5, 10 mM MgCl₂, 10 mM DTT, 5 mM ATP and 25 µg/mL BSA. The ligation mix can be selected from the reagent in the kit under Cat. # M0367S from NEB.

In some cases, the kit further comprised a tube 3, a tube 4, a tube 5, etc., e.g., tube 3 comprising a PCR reaction reagent, tube 4 comprising an adapter, and tube 5 comprising a primer. The adapter may be, for example, an adapter 1 (D9-T) packaged in a separate tube for constructing a third-generation sequencing library, or an adapter 2 packaged in a separate tube for constructing a next-generation sequencing library e.g., for ILLUMINA platform; the PCR amplification reagent can be selected from a common PCR amplification kit (comprising Taq enzyme) and a high-fidelity PCR amplification kit (comprising a high-fidelity enzyme, such as pfu), and can be selected from PCR amplification kits from Thermo Fisher, Merck, Beckman, etc.

With the kit, libraries suitable for various sequencing platforms, including but not limited to next-generation sequencing platforms and third-generation sequencing platforms, can be efficiently constructed.

This example provides use of the kit of Example 1 in library construction. In this example, 4 parallel experiments were conducted, and the samples used were F4, F5, F6 and F7 genomes. Before the genomic DNA fragmentation, the genome required a preliminary quality assessment to ensure that the genome was present in a solvent free of metal ion chelating agents or other salts, as well as a gel electrophoresis to check the genome integrity. The specific steps of library construction were as follows:

### 1. Genomic DNA fragmentation, end-repair and dA-tailing

In this step, DNAs were fragmented, filled-in at their ends, phosphorylated at 5'-ends, and dA-tailed at 3'-ends.

The reaction system was prepared in a PCR tube, as shown in Table 1:

**Table 1**

| | |
|---|---|
| H₂O | 14 µL |
| End Prep Mix | 4µL |
| DNA(25ng/ µL | 2 µL |
| Total | 20 µL |

The PCR tube was placed in a PCRsystem, and the reaction conditions were set as shown in Table 2:

**Table 2**

| Temp | Time |
|---|---|
| Heated lid at 105 °C | On |
| 32° C | 15-20min |
| 65° C | 10min |
| 4° C | Hold |

After the reaction was completed, the next step was immediately performed.

### 2. Adapter ligation

After the reaction in step 1 was completed, an adapter ligation system was prepared in a PCR tube, as shown in Table 3:

**Table 3**

| Component | Volume |
|---|---|
| Product from step 1 | 20 µL |
| Adapter 1 (15 µM) | 5 µL |
| Ligation Mix (NEB M0367S) | 25 µL |
| Total | 50 µL |

The reaction conditions: left to stand at room temperature for 15 min.

### 3. Purification

The reaction product was purified using VAHTS^{™} DNA Clean Beads according to the following steps:
1) The adapter-ligated system was transferred to a 1.5 mL EP tube, and 0.8× (40 µL) magnetic beads were added. The mixture was pipetted 10 times to be well mixed and left to stand at room temperature for 3 min.
2) The 1.5 mL EP tube was placed on a magnetic rack for 2-3 min, and the supernatant was removed.
3) With the EP tube kept on the rack, the magnetic beads was washed with freshly prepared 200 µL of 80% ethanol.
4) After ethanol was removed, the tube was briefly centrifuged, placed on the magnetic rack again and cleaned with a 10 µL pipette.
5) The lid was opened, and the magnetic beads were dried for 5-10 min until ethanol was completely volatilized.
6) The tube was taken out of the rack, and eluted with 22 µL of ddH₂O. After well mixing, the tube was left to stand at room temperature for 3 min, and then placed on the magnetic rack for 3 min. 20 µL of the product was recovered, and 1.2× (24 µL) magnetic beads were added. The resulting mixture was pipetted 10 times to be well mixed and left to stand at room temperature for 3 min.
7) The 1.5 mL EP tube was placed on a magnetic rack for 2-3 min, and the supernatant was removed.
8) With the EP tube kept on the rack, the magnetic beads was washed with freshly prepared 200 µL of 80% ethanol.
9) After ethanol was removed, the tube was briefly centrifuged, placed on the magnetic rack again and cleaned with a 10 µL pipette.
10) The lid was opened, and the magnetic beads were dried for 5-10 min until ethanol was completely volatilized.
11) The tube was taken out of the rack, and eluted with 12 µL of ddH₂O. After well mixing, the tube was left to stand at room temperature for 3 min, and then placed on the magnetic rack for 3 min. 13 µL of the product was recovered.

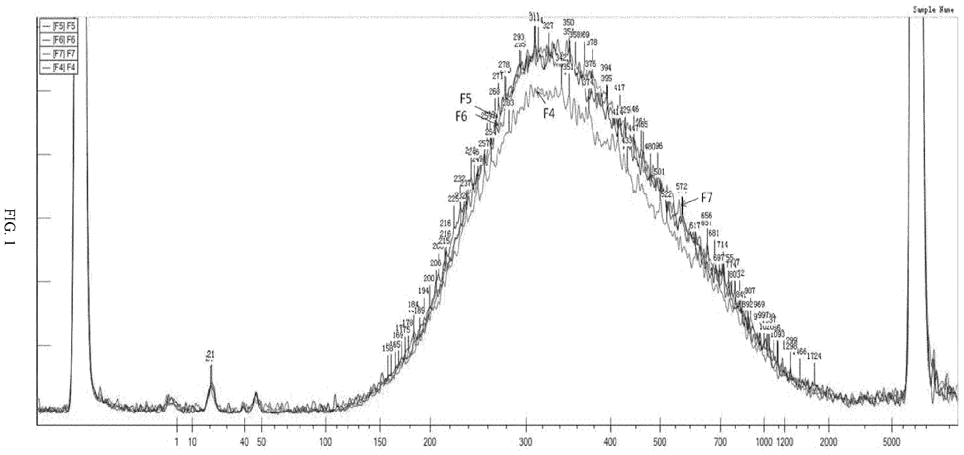

1 µL of the recovered product (ligation product) was subjected to labchip assay. The results are shown in FIG. 1, which shows that the main band of the 4 samples after adapter ligation was at 320 bp. From the results of the 4 samples, it can be seen that End Prep Mix can stably fragment the genomes into a specific size.

### 4. Library amplification

The recovered product was subjected to PCR amplification. The PCR amplification system was prepared as shown in Table 4:

**Table 4**

| Component | Volume |
|---|---|
| Amplification module | 12.5 µL |
| Ligation product | 11.5 µL |
| Forward primer (10 µM) | 0.5 µL |
| Reverse primer (10 µM) | 0.5 µL |
| Total | 25 µL |

The forward primer:
5'-AATGATACGGCGACCACCGAGATCTACACTCTTTCCCTACACGACGCTCTTCCGATCT-3'(SEQ ID NO: 6)

The reverse primer: 5'-GTGACTGGAGTTCAGACGTGTGCTCTTCCGATCT-3' (SEQ ID NO: 7)
The PCR amplification conditions are shown in Table 5:

**Table 5**

| Temperature (°C) | Time | Number of cycles |
|---|---|---|
| 95 | 1 min | 1 |
| 95 | 20 sec | 5-20 cycles |
| Tm-5 | 20 sec | |
| 72 | 30 sec | |
| 72 | 5 min | 1 |
| 4 °C | Hold | |

### 5. Purification

The reaction product was purified using VAHTS DNA Clean Beads.
(1) After the magnetic beads were equilibrated to room temperature, VAHTS DNA Clean Beads were vortexed to be well mixed.
(2) The ligated PCR system (25 µL) was transferred to a 1.5 mL EP tube, and 1.8× (45 µL) magnetic beads were added.
   The resulting mixture was pipetted 10 times to be well mixed and left to stand at room temperature for 3 min.
(3) The 1.5 mL EP tube was placed on a magnetic rack for 2-3 min, and the supernatant was removed.
(4) With the EP tube kept on the rack, the magnetic beads was washed with freshly prepared 200 µL of 80% ethanol.
(5) Step 4 was repeated.
(6) After ethanol was removed, the tube was briefly centrifuged, placed on the magnetic rack again and cleaned with a 10 µL pipette.
(7) The lid was opened, and the magnetic beads were dried for 5-10 min until ethanol was completely volatilized.
(8) The 1.5 mL tube was taken out of the magnetic rack for elution: 22 µL of ddH₂O was added, and the resulting mixture was vortexed or gently pipetted to be well mixed, and left to stand at room temperature for 3 min. The EP tube was briefly centrifuged and placed on the magnetic rack. After the solution became clear, 20 µL of the supernatant was transferred to a new 1.5 mL EP tube.

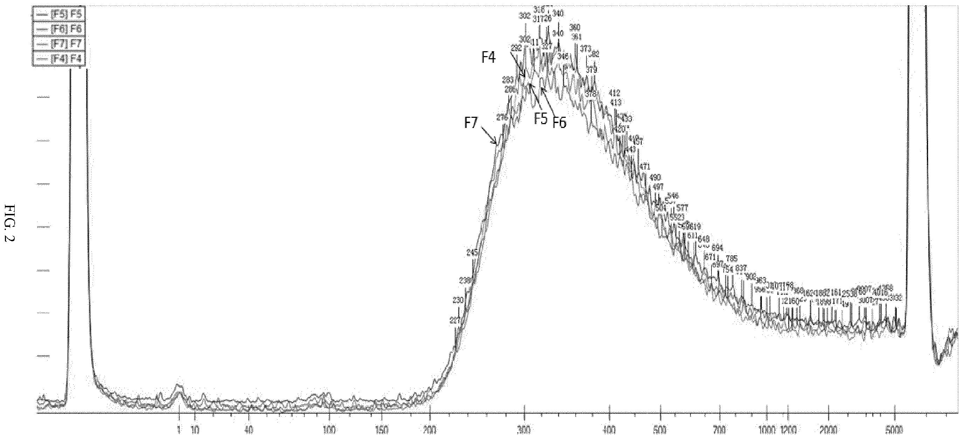

1 µL of the purified product after PCR amplification was subjected to labchip assay. The results are shown in FIG. 2, which shows that the main band of the products obtained by adapter ligation and then amplification of the 4 samples was at 320 bp. As the primer used was paired with the sequence in the adapter in a complementary way and the segment was effectively amplified according to the amplification results, the adapter can be effectively ligated using Ligation Mix, and the segment size was consistent after the 4 samples were fragmented and ligated to the adapter.

### 6. Quantitative on-line sequencing

The qualified library was quantified for sequencing on an Illumina sequencer.

In the related steps above, preferably, the amount of template DNA ranged from 15 ng to 50 ng, and OD260/OD280 = 1.8-2.0. The fragmentation size ranged from 150 bp to 200 bp.

The recommended adapter: Input DNA (molar ratio) was between 100:1 and 600:1. Excessive adapter input may result in adapter or adapter dimer residue, while insufficient adapter input would affect the ligation efficiency and thus result in reduced library yield. Adapter working concentration: 25 µM, template molar amount (pmol) ≈ Input DNA mass (ng)/[0.66*Input DNA average length (bp)].

The amount of magnetic beads directly affects the lower limit of the length of DNA that can be purified. The higher the multiplier, the smaller the lower limit of the length of DNA that can be purified; otherwise, the greater the lower limit of the length of DNA that can be purified. For example: 1× magnetic beads can only efficiently purify a DNA longer than 250 bp, and a shorter DNA may be lost in a large amount in the purification process; after an increase to 1.8×, a DNA of 150 bp can also be purified efficiently.

The eluted product can be stably stored at 4 °C for one week. When stored for a long time, the product should be kept at -20 °C to avoid unnecessary repeated freezing and thawing.

This example provides other use of the kit of Example 1 in library construction. In this example, 12 parallel experiments were conducted, and the samples used were N5, N6, N13, N14, N21, N22, N23, N24, O5, 06, O11 and 012 genomes. Before the genomic DNA fragmentation, the genome required a preliminary quality assessment to ensure that the genome was present in a solvent free of metal ion chelating agents or other salts, as well as a gel electrophoresis to check the genome integrity. The specific steps of library construction were as follows:

### 1. DNA fragmentation, end-repair and dA-tailing

In this step, DNAs were fragmented, filled-in at their ends, phosphorylated at 5'-ends, and dA-tailed at 3'-ends.

The reaction systems for the different samples were the same, as shown in table 6. The reaction times at 32 °C were different, and were 20 min, 22 min, 25 min, 30 min, 35 min and 40 min, respectively.

The sample types and corresponding reaction times were as follows:

| | | |
|---|---|---|
| N5: 50 ng, 32 °C | 20 min; N6: 50 ng, 32 °C | 20 min; |
| N13: 50 ng, 32 °C | 22 min; N14: 50 ng, 32 °C | 22 min; |
| N21: 50 ng, 32 °C | 25 min; N22: 50 ng, 32 °C | 25 min; |
| N23: 50 ng, 32 °C | 35 min; N24: 50 ng, 32 °C | 35 min; |
| O5: 50 ng, 32 °C | 30 min; O6: 50 ng, 32 °C | 30 min; |
| O11: 50 ng, 32 °C | 40 min; O12: 50 ng, 32 °C | 40 min. |

**Table 6**

| | |
|---|---|
| H₂O | 14 µL |
| End Prep Mix | 4 µL |
| DNA(25ng/ µL) | 2 µL |
| Total | 20 µL |

The PCR tube was placed in a PCR amplifier, and the reaction conditions were set as shown in Table 7:

**Table 7**

| Temp | Time |
|---|---|
| Heated lid at 105 °C | On |
| 32 °C | (20,22,25, 30,35,40)min |
| 65 °C | 10min |
| 4 °C | Hold |

After the reaction was completed, the next step was immediately performed.

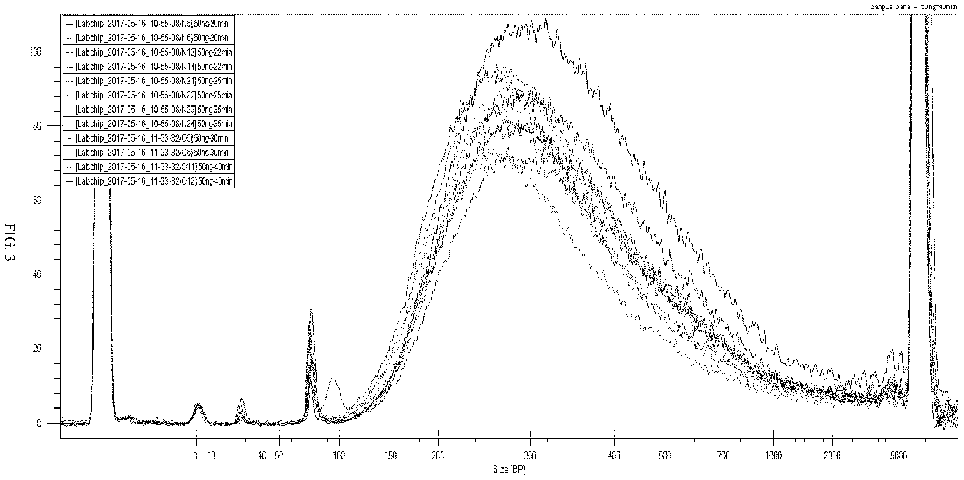

The product after the reaction was subject to Labchip assay, and the results are shown in FIG.3. In the curve, the highest main peaks to the lowest main peaks correspond to samples N5, N6, N13, N14, N21, N22, N23, N24, 05, 06, O11 and 012, respectively. As can be seen from FIG. 3, after fragmentation by the fragmentation enzyme at 32 °C for 20-40 min, the genomic DNAs were effectively fragmented, and the main band of the fragments is at about 280 bp; different fragmentation times had small effect on the fragmentation size of the genomes, with a fluctuation of about 20 bp.

### 2. Adapter ligation

After the reaction in step 1 was completed, an adapter ligation system was prepared in a PCR tube, as shown in Table 8:

**Table 8**

| Component | Volume |
|---|---|
| Product from step 1 | 20 µL |
| Adapter (D9-T) (15 µM) | 5 µL |
| Ligation Mix(NEB M0367S) | 25 µL |
| Total | 50 µL |

The reaction conditions: left to stand at room temperature for 15 min.

### 3. Purification

The reaction product was purified using VAHTS DNA Clean Beads according to the following steps:
12) The adapter-ligated system was transferred to a 1.5 mL EP tube, and 0.8× (40 µL) magnetic beads were added. The mixture was pipetted 10 times to be well mixed and left to stand at room temperature for 3 min.
13) The 1.5 mL EP tube was placed on a magnetic rack for 2-3 min, and the supernatant was removed.
14) With the EP tube kept on the rack, the magnetic beads was washed with freshly prepared 200 µL of 80% ethanol.
15) After ethanol was removed, the tube was briefly centrifuged, placed on the magnetic rack again and cleaned with a 10 µL pipette.
16) The lid was opened, and the magnetic beads were dried for 5-10 min until ethanol was completely volatilized.
17) The tube was taken out of the rack, and eluted with 22 µL of ddH₂O. After well mixing, the tube was left to stand at room temperature for 3 min, and then placed on the magnetic rack for 3 min. 20 µL of the product was recovered, and 1.2× (24 µL) magnetic beads were added. The resulting mixture was pipetted 10 times to be well mixed and left to stand at room temperature for 3 min.
18) The 1.5 mL EP tube was placed on a magnetic rack for 2-3 min, and the supernatant was removed.
19) With the EP tube kept on the rack, the magnetic beads was washed with freshly prepared 200 µL of 80% ethanol.
20) After ethanol was removed, the tube was briefly centrifuged, placed on the magnetic rack again and cleaned with a 10 µL pipette.
21) The lid was opened, and the magnetic beads were dried for 5-10 min until ethanol was completely volatilized.
22) The tube was taken out of the rack, and eluted with 12 µL of ddH₂O. After well mixing, the tube was left to stand at room temperature for 3 min, and then placed on the magnetic rack for 3 min. 10 µL of the product was recovered.

The recovered product can be stably stored at 4 °C for one week. When stored for a long time, the product should be kept at -20 °C to avoid unnecessary repeated freezing and thawing.

### 4. Sequence capture and on-line sequencing

The qualified library was quantified for sequence capture and sequencing using a single-molecule sequencer.

A probe (the sequence thereof is set forth in SEQ ID NO: 8) was immobilized on a chip, e.g., by using the method disclosed in the CN105112408A, and the prepared library was diluted with 3× SSC hybridization solution and then hybridized with the probe immobilized on the chip. The number of the adapter sequences hybridized with the probe was then determined according to Cy3 signals.
SEQ ID NO: 8: 5'-TTTTTTTTTTTCCTTGATACCTGCGACCATCCAGTTCCACTCAGATGTGTATAAGAGACAG-3'.

The procedure for library-chip hybridization was as follows:
(1) Chip selection: the substrate glass of the chip used was an epoxy-modified glass chip from SCHOTT; a probe set forth in SEQ ID NO: 4 was immobilized by reacting amino groups on the probe with epoxy groups on the chip surface, e.g., by the method disclosed in CN109610006A, with the immobilized probe density being 18000 Dot/FOV in a region of 110×110 µm, i.e., there were 18000 dots in a 110×110 µm field of view.
(2) Hybridization solution preparation: as shown in Table 9, the hybridization solution was prepared from 20× SSC buffer (Sigma, # S6639-1L) having a final concentration of 3× SSC and a library having a concentration of 1 nM, with a total volume of 40 µL. The prepared hybridization solution was denatured at 95 °C for 2 min, and then quickly transferred onto ice for cooling.

**Table 9**

| | |
|---|---|
| 20× SSC buffer | 6 µL |
| Library | Final concentration of 1 nM |
| Nucleic acid-free water | Making up to 40 µL |

(3) The denatured hybridization solution was quickly loaded onto the chip, which was then left to stand at 55 °C for 30 min to allow hybridization of the library with the probe on the chip surface.
(4) The chip was washed with 3× SSC, 1× SSC and 0.1× SSC in sequence.

The library obtained by hybrid capture was sequenced using the third-generation sequencing platform GenoCare^{™}.

## Claims

1. A method for processing a DNA, comprising fragmenting, end-repairing and dA-tailing the DNA in a first pre-mixed system to obtain a first product, wherein the first pre-mixed system comprises a pre-mixed enzyme comprising a DNA-fragmentation enzyme, a polynucleotide kinase and a DNA polymerase.

2. The method according to claim 1, wherein the DNA has a size of no less than 5 Kbp; optionally,
wherein the first product has a size of 100-400 bp, optionally 100-300 bp.

3. The method according to claim 1, wherein the DNA in the first pre-mixed system has an amount of 10-60 ng, optionally 15-50 ng.

4. The method according to any of claims 1 to 3, wherein the first pre-mixed system further comprises Mg²⁺, wherein the Mg²⁺ has a concentration of 60-100 mM, optionally 60-75 mM.

5. The method according to claim 4, wherein the DNA polymerase is selected from at least one of DNA polymerase I and Taq DNA polymerase; optionally
wherein the DNA polymerase consists of Klenow fragment and Taq DNA polymerase; optionally
wherein the polynucleotide kinase is T4 polynucleotide kinase.

6. The method according to any of claims 1 to 5, wherein the amount of DNA-fragmentation enzyme in the first pre-mixed system is at least 2 times the amount of the polynucleotide kinase enzyme therein, wherein the amount corresponds to the enzyme activity.

7. The method according to claim 6, wherein the ratio of the amount of the DNA-fragmentation enzyme, the polynucleotide kinase, Klenow fragment and Taq DNA polymerase in the first pre-mixed system is [16, 35]:[6, 10]:[1, 3]:[1, 5], wherein the ratio of the amount corresponds to the ratio of the enzyme activity.

8. The method according to any of claims 1 to 5, further comprising providing at least one end of the first product with a predetermined sequence in a second pre-mixed system to obtain a second product, wherein the second pre-mixed system comprises the first pre-mixed system and the predetermined sequence;
wherein optionally the second pre-mixed system further comprises a ligase, wherein optionally the ligase is T4 DNA ligase.

9. The method according to claim 8, wherein the predetermined sequence is a double-stranded DNA consisting of a first strand and a second strand that are complementary, and has at least one single-stranded end;
wherein optionally the 5'-end of the first strand has a phosphate group;
wherein optionally the 3'-end of the second strand has no hydroxyl group.

10. The method according to claim 9, wherein 80 °C ≥ (Tm1 - Tm2) ≥ 10 °C and 90 °C ≥ Tm1 ≥ 50 °C, wherein Tm1 is a melting temperature of the first strand, and Tm2 is a melting temperature of the second strand;
wherein optionally the Tm1 is 71 °C, and the Tm2 is 45.6 °C; optionally
wherein the predetermined sequence has a sequence selected from at least one of the following combinations:
1) SEQ ID NO: 1 and SEQ ID NO: 2;
2) SEQ ID NO: 1 and SEQ ID NO: 3; and
3) SEQ ID NO: 4 and SEQ ID NO: 5.

11. A method for sequencing a DNA, comprising:
processing the DNA by the method according to any of claims 1 to 7 to obtain the first product;
processing the first product by the method according to any of claims 8 to 10 to obtain the second product; and
linking the second product to a surface of a solid substrate and sequencing the second product to determine at least part of the sequence of the DNA;
wherein optionally the second product has an optically detectable label,
wherein optionally the second product has a fluorescent molecule.

12. The method according to claim 11, wherein the solid substrate has a probe on the surface thereof, and the second product is linked to the surface of the solid substrate by ligating the probe, wherein the probe has a length of 20-80 nt; optionally
wherein the predetermined sequence is a double-stranded DNA consisting of a first strand and a second strand that are complementary, and has a single-stranded end located on the first strand; optionally
wherein the first strand is fully complementary to the probe, and the length of the first strand is not longer than the length of the probe; optionally
the method further comprising denaturing the second product before linking the second product to the surface of the solid substrate.

13. A kit for implementing the method according to any of claims 1 to 10 or for implementing the method according to claim 11 or 12, comprising the first pre-mixed system of the method of any of claims 1 to 10.

14. The kit according to claim 13, further comprising the second pre-mixed system of the method of any of claims 8 to 10.

15. The kit according to claim 14, further comprising a probe linkable to a surface of a solid substrate, wherein the probe has a length of 20-80 nt; optionally
wherein the predetermined sequence is a double-stranded DNA consisting of a first strand and a second strand that are complementary, and has a single-stranded end located on the first strand, and the length of the first strand is not longer than the length of the probe.

## Patentansprüche

1. Verfahren zum Verarbeiten einer DNA, umfassend das Fragmentieren, Reparieren des Endes und Versehen der DNA mit einem dA-Schwanz in einem ersten vorgemischten System, um ein erstes Produkt zu erhalten, wobei das erste vorgemischte System ein vorgemischtes Enzym umfasst, das ein DNA-Fragmentierungsenzym, eine Polynukleotidkinase und eine DNA-Polymerase umfasst.

2. Verfahren nach Anspruch 1, wobei die DNA eine Größe von mindestens 5 Kbp aufweist; gegebenenfalls
wobei das erste Produkt eine Größe von 100-400 bp, gegebenenfalls 100-300 bp aufweist.

3. Verfahren nach Anspruch 1, wobei die DNA in dem ersten vorgemischten System eine Menge von 10-60 ng, gegebenenfalls 15-50 ng aufweist.

4. Verfahren nach einem der Ansprüche 1 bis 3, wobei das erste vorgemischte System ferner Mg²⁺ umfasst, wobei das Mg²⁺ eine Konzentration von 60-100 mM, gegebenenfalls 60-75 mM aufweist.

5. Verfahren nach Anspruch 4, wobei die DNA-Polymerase ausgewählt ist aus mindestens einem von DNA-Polymerase I und Taq-DNA-Polymerase; gegebenenfalls
wobei die DNA-Polymerase aus Klenow-Fragment und Taq-DNA-Polymerase besteht; gegebenenfalls
wobei die Polynukleotidkinase die T4-Polynukleotidkinase ist.

6. Verfahren nach einem der Ansprüche 1 bis 5, wobei die Menge des DNA-Fragmentierungsenzyms in dem ersten vorgemischten System mindestens das Zweifache der Menge des Polynukleotidkinaseenzyms darin beträgt, wobei die Menge der Enzymaktivität entspricht.

7. Verfahren nach Anspruch 6, wobei das Verhältnis der Menge des DNA-Fragmentierungsenzyms, der Polynukleotidkinase, des Klenow-Fragments und der Taq-DNA-Polymerase in dem ersten vorgemischten System [16, 35]:[6, 10]:[1, 3]:[1, 5] beträgt, wobei das Verhältnis der Menge dem Verhältnis der Enzymaktivität entspricht.

8. Verfahren nach einem der Ansprüche 1 bis 5, ferner umfassend das Versehen von mindestens einem Ende des ersten Produkts mit einer vorbestimmten Sequenz in einem zweiten vorgemischten System, um ein zweites Produkt zu erhalten, wobei das zweite vorgemischte System das erste vorgemischte System und die vorbestimmte Sequenz umfasst;
wobei das zweite vorgemischte System gegebenenfalls ferner eine Ligase umfasst;
wobei die Ligase gegebenenfalls T4-DNA-Ligase ist.

9. Verfahren nach Anspruch 8, wobei die vorbestimmte Sequenz eine doppelsträngige DNA ist, die aus einem ersten Strang und einem zweiten Strang besteht, die komplementär sind, und mindestens ein einzelsträngiges Ende aufweist; wobei das 5'-Ende des ersten Strangs gegebenenfalls eine Phosphatgruppe aufweist;
wobei das 3'-Ende des zweiten Strangs gegebenenfalls keine Hydroxylgruppe aufweist.

10. Verfahren nach Anspruch 9, wobei 80 °C ≥ (Tm1 - Tm2) ≥ 10 °C und 90 °C ≥ Tm1 ≥ 50 °C, wobei Tm1 eine Schmelztemperatur des ersten Strangs und Tm2 eine Schmelztemperatur des zweiten Strangs ist;
wobei gegebenenfalls die Tm1 71 °C beträgt und die Tm2 45,6 °C beträgt; gegebenenfalls
wobei die vorbestimmte Sequenz eine Sequenz aufweist, die aus mindestens einer der folgenden Kombinationen ausgewählt ist:
1) SEQ ID NO: 1 und SEQ ID NO: 2;
2) SEQ ID NO: 1 und SEQ ID NO: 3; und
3) SEQ ID NO: 4 und SEQ ID NO: 5.

11. Verfahren zum Sequenzieren einer DNA, umfassend:
Verarbeiten der DNA durch das Verfahren nach einem der Ansprüche 1 bis 7, um das erste Produkt zu erhalten;
Verarbeiten des ersten Produkts durch das Verfahren nach einem der Ansprüche 8 bis 10, um das zweite Produkt zu erhalten; und
Binden des zweiten Produkts an eine Oberfläche eines festen Substrats und Sequenzieren des zweiten Produkts, um mindestens einen Teil der Sequenz der DNA zu bestimmen;
wobei das zweite Produkt gegebenenfalls eine optisch nachweisbare Markierung aufweist,
wobei das zweite Produkt gegebenenfalls ein fluoreszierendes Molekül aufweist.

12. Verfahren nach Anspruch 11, wobei das feste Substrat eine Sonde auf der Oberfläche davon aufweist und das zweite Produkt an die Oberfläche des festen Produkts gebunden wird, indem die Sonde ligiert wird, wobei die Sonde eine Länge von 20-80 nt aufweist; gegebenenfalls
wobei die vorbestimmte Sequenz eine doppelsträngige DNA ist, die aus einem ersten Strang und einem zweiten Strang besteht, die komplementär sind, und ein einzelsträngiges Ende aufweist, das sich am ersten Strang befindet; gegebenenfalls wobei der erste Strang vollständig komplementär zur Sonde ist und die Länge des ersten Strangs nicht länger als die Länge der Sonde ist; gegebenenfalls
wobei das Verfahren ferner das Denaturieren des zweiten Produkts umfasst, bevor das zweite Produkt an die Oberfläche des festen Substrats gebunden wird.

13. Kit zum Umsetzen des Verfahrens nach einem der Ansprüche 1 bis 10 oder zum Umsetzen des Verfahrens nach Anspruch 11 oder 12, umfassend das erste vorgemischte System des Verfahrens nach einem der Ansprüche 1 bis 10.

14. Kit nach Anspruch 13, ferner umfassend das zweite vorgemischte System des Verfahrens nach einem der Ansprüche 8 bis 10.

15. Kit nach Anspruch 14, ferner umfassend eine Sonde, die an die Oberfläche eines festen Substrats gebunden werden kann, wobei die Sonde eine Länge von 20-80 nt aufweist; gegebenenfalls
wobei die vorbestimmte Sequenz eine doppelsträngige DNA ist, die aus einem ersten Strang und einem zweiten Strang besteht, die komplementär sind, und ein einzelsträngiges Ende aufweist, das sich am ersten Strang befindet; und die Länge des ersten Strangs nicht länger als die Länge der Sonde ist.

## Revendications

1. Procédé de traitement d'un ADN, comprenant la fragmentation de l'ADN, la réparation d'extrémité de l'ADN et l'addition d'une queue dA à l'ADN dans un premier système pré-mélangé pour obtenir un premier produit, dans lequel le premier système pré-mélangé comprend une enzyme pré-mélangée comprenant une enzyme de fragmentation de l'ADN, une polynucléotide kinase et une ADN polymérase.

2. Procédé selon la revendication 1, dans lequel l'ADN a une taille non inférieure à 5 kpb ; éventuellement,
dans lequel le premier produit a une taille de 100 à 400 pb, éventuellement de 100 à 300 pb.

3. Procédé selon la revendication 1, dans lequel l'ADN dans le premier système pré-mélangé a une quantité de 10 à 60 ng, éventuellement de 15 à 50 ng.

4. Procédé selon l'une quelconque des revendications 1 à 3, dans lequel le premier système pré-mélangé comprend en outre du Mg²⁺, le Mg²⁺ ayant une concentration de 60 à 100 mM, éventuellement de 60 à 75 mM.

5. Procédé selon la revendication 4, dans lequel l'ADN polymérase est choisie parmi au moins une parmi l'ADN polymérase I et l'ADN polymérase Taq ; éventuellement dans lequel l'ADN polymérase consiste en le fragment de Klenow et l'ADN polymérase Taq ; éventuellement
dans lequel la polynucléotide kinase est la polynucléotide kinase T4.

6. Procédé selon l'une quelconque des revendications 1 à 5, dans lequel la quantité d'enzyme de fragmentation de l'ADN dans le premier système pré-mélangé est d'au moins deux fois la quantité d'enzyme polynucléotide kinase dans celui-ci, la quantité correspondant à l'activité enzymatique.

7. Procédé selon la revendication 6, dans lequel le rapport entre les quantités d'enzyme de fragmentation de l'ADN, de polynucléotide kinase, de fragment de Klenow et d'ADN polymérase Taq dans le premier système pré-mélangé est de [16, 35]:[6, 10]:[1, 3]:[1, 5], le rapport entre les quantités correspondant au rapport entre les activités enzymatiques.

8. Procédé selon l'une quelconque des revendications 1 à 5, comprenant en outre le fait de fournir à au moins une extrémité du premier produit une séquence prédéterminée dans un deuxième système pré-mélangé pour obtenir un deuxième produit, le deuxième système pré-mélangé comprenant le premier système pré-mélangé et la séquence prédéterminée ;
dans lequel le deuxième système pré-mélangé comprend éventuellement en outre une ligase, la ligase étant éventuellement l'ADN ligase T4.

9. Procédé selon la revendication 8, dans lequel la séquence prédéterminée est un ADN double brin consistant en un premier brin et un deuxième brin qui sont complémentaires, et comporte au moins une extrémité simple brin ;
dans lequel l'extrémité 5' du premier brin comporte éventuellement un groupe phosphate ;
dans lequel l'extrémité 3' du deuxième brin ne comporte éventuellement pas de groupe hydroxyle.

10. Procédé selon la revendication 9, dans lequel 80 °C ≥ (Tm1 - Tm2) ≥ 10 °C et 90 °C ≥ Tm1 ≥ 50 °C, Tm1 étant une température de fusion du premier brin, et Tm2 une température de fusion du deuxième brin ;
dans lequel, éventuellement, la Tm1 est de 71 °C et la Tm2 de 45,6 °C ; éventuellement
dans lequel la séquence prédéterminée a une séquence choisie parmi au moins une des combinaisons suivantes :
1) SEQ ID NO : 1 et SEQ ID NO : 2,
2) SEQ ID NO : 1 et SEQ ID NO : 3 ; et
3) SEQ ID NO : 4 et SEQ ID NO : 5.

11. Procédé de séquençage d'un ADN, comprenant :
le traitement de l'ADN par le procédé selon l'une quelconque des revendications 1 à 7 pour obtenir le premier produit ;
le traitement du premier produit par le procédé selon l'une quelconque des revendications 8 à 10 pour obtenir le deuxième produit ; et
la liaison du deuxième produit à une surface d'un substrat solide et le séquençage du deuxième produit pour déterminer au moins une partie de la séquence de l'ADN ;
dans lequel le deuxième produit comporte éventuellement une étiquette optiquement détectable,
dans lequel le deuxième produit comporte éventuellement une molécule fluorescente.

12. Procédé selon la revendication 11, dans lequel le substrat solide comporte une sonde à sa surface, et le deuxième produit est lié à la surface du substrat solide par ligature de la sonde, la sonde ayant une longueur de 20 à 80 nt ; éventuellement
dans lequel la séquence prédéterminée est un ADN double brin consistant en un premier brin et un deuxième brin qui sont complémentaires, et comporte une extrémité simple brin située sur le premier brin ; éventuellement
dans lequel le premier brin est totalement complémentaire de la sonde, et la longueur du premier brin n'est pas supérieure à la longueur de la sonde ; éventuellement
le procédé comprenant en outre la dénaturation du deuxième produit avant la liaison du deuxième produit à la surface du substrat solide.

13. Kit pour la mise en oeuvre du procédé selon l'une quelconque des revendications 1 à 10 ou pour la mise en oeuvre du procédé selon la revendication 11 ou 12, comprenant le premier système pré-mélangé du procédé selon l'une quelconque des revendications 1 à 10.

14. Kit selon la revendication 13, comprenant en outre le deuxième système pré-mélangé du procédé selon l'une quelconque des revendications 8 à 10.

15. Kit selon la revendication 14, comprenant en outre une sonde pouvant être liée à une surface d'un substrat solide, la sonde ayant une longueur de 20 à 80 nt ; éventuellement
dans lequel la séquence prédéterminée est un ADN double brin consistant en un premier brin et un deuxième brin qui sont complémentaires, et comporte une extrémité simple brin située sur le premier brin ; et la longueur du premier brin n'est pas supérieure à la longueur de la sonde.
